# EUROPEAN PATENT SPECIFICATION

(11) **EP 2 603 486 B1**
(45) Date of publication and mention of the grant of the patent: **17.12.2014**
(21) Application number: 11743536.2
(22) Date of filing: 11.08.2011
(51) Int. Cl.: C07C 235/08, A61K 31/164

(54) **PANTHENYL DOCOSAHEXAENEOATE AND ITS USE FOR TREATING AND PREVENTING CARDIOVASCULAR DISEASES**
PANTHENYLDOCOSAHEXAENOAT UND SEINE VERWENDUNG ZUR BEHANDLUNG UND VORBEUGUNG VON HERZ-KREISLAUF-ERKRANKUNGEN
DOCOSAHEXANOATE DE PANTHÉNYLE ET SON UTILISATION POUR TRAITER ET PRÉVENIR LES MALADIES CARDIOVASCULAIRES

(30) Priority: 11.08.2010 FR 1056560
(43) Date of publication of application: 19.06.2013
(73) Proprietor: Pierre Fabre Médicament, 92100 Boulogne-Billancourt (FR)
(72) Inventor: LANTOINE-ADAM, Frédérique, F-31300 Toulouse (FR); LETIENNE, Robert, F-81100 Castres (FR); DUPONT-PASSELAIGUE, Elisabeth, F-81100 Castres (FR)
(74) Representative: Regimbeau
(86) International application number: PCT/EP2011/063854
(87) International publication number: WO 2012/020094

(56) References cited:
- WO-A1-2011/018480
- WO-A2-2007/147899
- GB-A- 1 170 132

## Description

The present invention relates to a monoester of docosahexaenoic acid (DHA) with panthenol having particular properties, notably as a drug in the treatment and the prevention of cardiovascular diseases.

Polyunsaturated fatty acids of the Omega-3 series, in particular docosahexaenoic acid, are known for their potential use in the treatment of certain cardiovascular diseases and the modulation of corresponding risk factors. In particular, they are known in the treatment of hyperlipidemia, hypercholesterolemia and hypertension. Clinical trials conducted with formulations containing a high concentration of DHA ethyl ester on patients who had suffered a myocardial infarction showed their effectiveness by reducing mortality, in particular sudden death. These results were partly attributed to a stabilizing effect on the cell membranes of ventricular cardiomyocytes, which prevents the appearance of malignant arrhythmia in the presence of ischemic myocytes among patients having suffered an infarction or in experimental models which reproduce such conditions.

Furthermore, it is known according to the patent application WO2004/047835 that DHA ethyl esters can be used to prevent atrial fibrillation.

GB1170132 describes di- or triesters of shorter acids with Pantothenol and their pharmaceutical uses.

In addition, WO2007/147899 describes the preparation and the use of certain DHA esters, in particular the pharmaceutical effects of a particular DHA ester, pyridin-3-ylmethyl docosahexaenoate (nicotinyl alcohol DHA ester).

However, the present Inventors discovered that, in a surprising manner, a particular monoester of DHA with panthenol possessed particularly advantageous properties in the context of the treatment of cardiovascular diseases.

Panthenol is the alcohol analog of pantothenic acid, more commonly known as vitamin B5. In the body, panthenol is transformed into pantothenic acid which then becomes a large part of the compound "coenzyme A," which is of particular interest in cell metabolism. Indeed, it takes part in the metabolism of lipids, carbohydrates and proteins. Panthenol also participates in the formation of acetylcholine and steroids of the adrenal gland. It also intervenes in detoxication of foreign bodies and in resistance to infections.

In a surprising manner, the Inventors discovered that the administration to pigs of the panthenyl docosahexaenoate of the following formula: 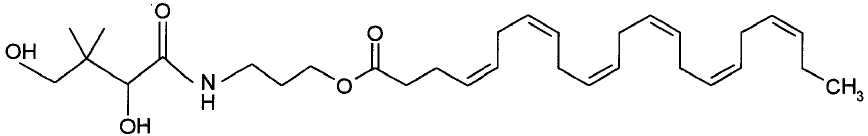 made it possible to significantly increase the duration of the atrial refractory period (see example 2 of the present application) compared to other DHA esters and in particular compared to the diester of panthenol and DHA.

The present invention thus relates to the ester of docosahexaenoic acid with panthenol, or panthenyl docosahexaenoate, of the following formula: 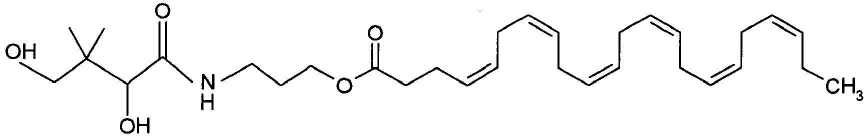 or a pharmaceutically acceptable salt, enantiomer or diastereoisomer of same, or a mixture thereof, including a racemic mixture.

In other words, the present invention relates to (2,4-dihydroxy-3,3-dimethylbutanamido)propyl-docosa-4,7,10,13,16,19-hexanoate or a pharmaceutically acceptable salt, enantiomer or diastereoisomer of same, or a mixture thereof, including a racemic mixture.

In the present invention, the term "enantiomers" refers to optical isomer compounds which have identical molecular formulas but which differ by their spatial configuration and which are non-superimposable mirror images of each other. The term "diastereoisomers" refers to optical isomers which are not mirror images of each other. In the context of the present invention, a "racemic mixture" is a mixture with equal proportions of the levorotatory and dextrorotatory enantiomers of a chiral molecule.

In the present invention, the term "pharmaceutically acceptable" refers to that which is useful in the preparation of a pharmaceutical composition that is generally safe, non-toxic and neither biologically nor otherwise undesirable and that is acceptable for veterinary use as well as for use in human pharmaceuticals.

The term "pharmaceutically acceptable salts" of a compound refers to salts that are pharmaceutically acceptable, as defined herein, and that possess the desired pharmacological activity of the parent compound. Such salts include:
(1) acid addition salts formed with mineral acids such as hydrochloric acid, hydrobromic acid, sulfuric acid, nitric acid, phosphoric acid and the like; or formed with organic acids such as acetic acid, benzenesulfonic acid, benzoic acid, camphorsulfonic acid, citric acid, ethane-sulfonic acid, fumaric acid, glucoheptonic acid, gluconic acid, glutamic acid, glycolic acid, hydroxynaphthoic acid, 2-hydroxyethanesulfonic acid, lactic acid, maleic acid, malic acid, mandelic acid, methanesulfonic acid, muconic acid, 2-naphthalenesulfonic acid, propionic acid, salicylic acid, succinic acid, dibenzoyl-L-tartaric acid, tartaric acid, p-toluenesulfonic acid, trimethylacetic acid, trifluoroacetic acid and the like; or
(2) salts formed when an acid proton present in the parent compound either is replaced by a metal ion, for example an alkaline metal ion, an alkaline-earth metal ion or an aluminum ion; or coordinates with an organic or inorganic base. Acceptable organic bases include diethanolamine, ethanolamine, N-methylglucamine, triethanolamine, tromethamine and the like. Acceptable inorganic bases include aluminum hydroxide, calcium hydroxide, potassium hydroxide, sodium carbonate and sodium hydroxide.

Preferred pharmaceutically acceptable salts are the salts formed from hydrochloric acid, trifluoroacetic acid, dibenzoyl-L-tartaric acid and phosphoric acid.

It should be understood that all references to pharmaceutically acceptable salts include solvent addition forms (solvates) or crystalline forms (polymorphs) as defined herein, as well as acid addition salts.

In a particular embodiment, the inventive ester is panthenyl docosahexaenoate, or "D-panthenol DHA ester," of following formula A: 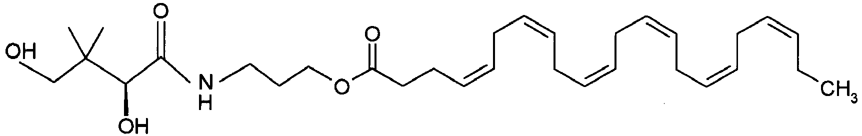 or a pharmaceutically acceptable salt, enantiomer or diastereoisomer of same, or a mixture thereof, including a racemic mixture.

A method for synthesizing this particular compound is proposed in example 1 of the present application.

The present invention also relates to a method for preparing the panthenol ester of the present invention, by esterification of docosahexaenoic acid with panthenol, for example with D-panthenol, comprising the following steps:
a) Selective protection of two OH functional groups of panthenol, in particular of D-panthenol, by an O-protective group, advantageously by trimethylchlorosilane,
b) Esterification of the unprotected OH functional group by DHA in the presence of 1-[(1H-imidazol-1-yl)carbonyl]-1H-imidazol and N, N-dimethylpyridin-4-amine,
c) Deprotection of the two protected OH functional groups.

The deprotection of step c) is well known to those persons skilled in the art, and can be carried out, for example, in methanol and p-toluenesulfonic acid when the O-protective group is trimethylchlorosilane.

In the context of the present invention, the term "O-protective group" refers to any substituent that protects the hydroxyl group against undesirable reactions during the preparation of the monoester, such as the O-protective groups described in Greene, "Protective Groups in Organic Synthesis" (John Wiley & Sons, New York (1981)) and Harrison et al. "Compendium of Synthetic Organic Methods", Vols. 1 to 8 (J. Wiley & Sons, 1971 to 1996).

The present invention also relates to a pharmaceutical composition comprising the ester of DHA with panthenol of the present invention, for example the D-panthenol DHA ester of formula A of the present invention, and at least one pharmaceutically acceptable excipient.

The pharmaceutical compositions of the present invention can be formulated for administration in mammals, including man. Dosing varies according to the treatment and the disease in question. These compositions are prepared in such a way as to be administered by oral, sublingual, subcutaneous, intramuscular, intravenous, transdermal, local or rectal route. In this case, the active ingredient can be administered in unit-dose forms, in mixture with traditional pharmaceutical excipients, to animals or to humans. Suitable unit-dose administration forms include oral-route forms such as tablets, gelatin capsules, powders, granules and oral solutions or suspensions, sublingual and buccal administration forms, subcutaneous, topical, intramuscular, intravenous, intranasal or intraocular administration forms and rectal administration forms.

When a solid composition is prepared in tablet form, the primary active ingredient is mixed with a pharmaceutical carrier such as gelatin, starch, lactose, magnesium stearate, talc, gum arabic, silica or analogues. Tablets can be coated with sucrose or other suitable materials or they can be treated in such a way that they have delayed or extended activity and that they continuously release a predetermined quantity of the active ingredient.

A gelatin capsule preparation is obtained by mixing the active ingredient with a diluent and then pouring the mixture obtained into soft or hard gelatin capsules.

A preparation in syrup or elixir form can contain the active ingredient in conjunction with a sweetener, an antiseptic, as well as a flavoring agent and a suitable coloring agent.

Powders or granules that can be dispersed in water can contain the active ingredient in a mixture with dispersion agents, wetting agents or suspension agents, as well as with taste correctors or sweeteners.

Suppositories, which are prepared with binders that melt at rectal temperature, such as cocoa butter or polyethylene glycol, for example, are used for rectal administration.

Aqueous suspensions, isotonic saline solutions or sterile injectable solutions containing pharmacologically-compatible dispersion agents and/or wetting agents can be used for parenteral (intravenous, intramuscular, etc.), intranasal or intraocular administration.

The active ingredient can also be formulated in the form of microcapsules, optionally with one or more additives.

Advantageously, the pharmaceutical composition of the present invention is intended for administration by oral or intravenous route, advantageously by intravenous route in the case of post-infarction treatment.

In this case, the pharmaceutical composition advantageously contains a polyoxyethylene fatty acid, such as Solutol HS 15, and at least one phospholipid derivative such as that described in application FR0955612.

The pharmaceutical composition of the present invention can include other active ingredients that give rise to a complementary or possibly synergistic effect.

The present invention also relates to the docosahexaenoic acid ester of the present invention, *i.e.,* panthenyl docosahexaenoate, and in particular the panthenyl docosahexaenoate of formula A, or the pharmaceutical composition of the present invention for the use of same as a drug.

The present invention also relates to the docosahexaenoic acid ester of the present invention, *i.e.,* panthenyl docosahexaenoate, and in particular the panthenyl docosahexaenoate of formula A, or the pharmaceutical composition of the present invention for the use of same as a drug intended for the prevention and/or treatment of cardiovascular disease, advantageously selected from atrial and/or ventricular arrhythmia, tachycardia and/or fibrillation; for the prevention and/or treatment of diseases represented by defects in electrical conduction in myocardial cells; for the prevention and/or treatment of multiple risk factors for cardiovascular disease, advantageously selected from hypertriglyceridemia, hypercholesterolemia, hypertension, notably arterial hypertension, in particular refractory arterial hypertension, hyperlipidemia, dyslipidemia, advantageously mixed dyslipidemia, and/or factor VII hyperactivity in blood coagulation; for the treatment and/or primary or secondary prevention of cardiovascular disease derived from auricular and/or ventricular arrhythmia, tachycardia, fibrillation and/or electrical conduction defects induced by myocardial infarction, advantageously sudden death; and/or for post-infarction treatment.

In other words, the present invention relates to the docosahexaenoic acid ester of the present invention, *i.e*., panthenyl docosahexaenoate, and in particular the panthenyl docosahexaenoate of formula A, or the pharmaceutical composition of the present invention, for the use of same to prevent and/or treat the diseases cited above.

Advantageously, the present invention relates to the docosahexaenoic acid ester of the present invention, *i.e.,* panthenyl docosahexaenoate, in particular the panthenyl docosahexaenoate of formula A, or the pharmaceutical composition of the present invention, for the use of same as a drug intended for the prevention and/or the treatment of atrial fibrillation.

The invention will be better understood in reference to the figure and the examples which follow.

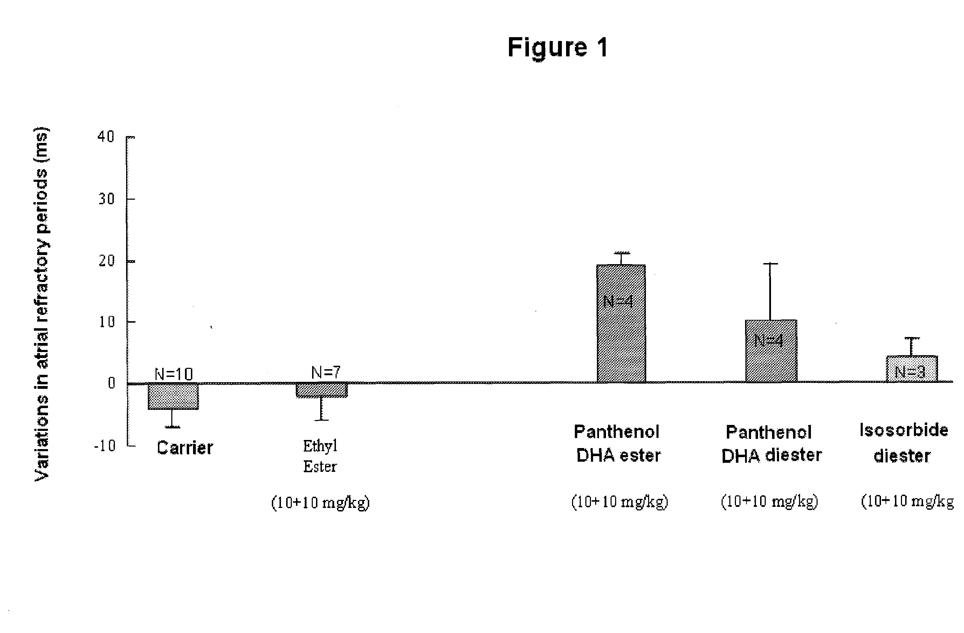

Figure 1 represents variations in refractory periods *in vivo* after administration of the carrier, or the DHA ethyl ester, versus the panthenyl docosahexaenoate of the invention, the diester of panthenol and DHA, and the diester of isosorbide and DHA, according to the protocol described in example 2 below.

The following examples are provided for illustrative purposes and are nonrestrictive.

### Example 1: Synthesis of the panthenyl docosahexaenoate of formula A

### 1. Synthesis of intermediate compound I derived from panthenol (protection of the alcohol functional groups on the left side of panthenol)

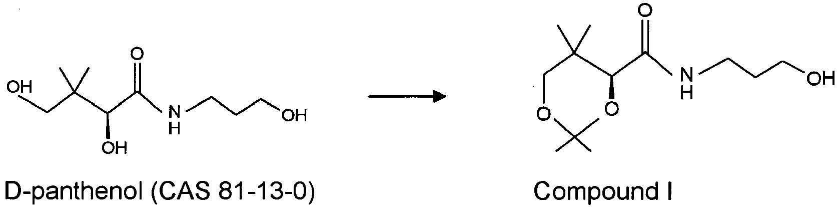

A 2000 ml three-neck flask, purged and maintained under a nitrogen atmosphere, is used to synthesize this compound.

120 ml of trimethylchlorosilane (TMCS) was added drop wise with stirring at a temperature of 10-15 °C to a solution of (2R)-2,4-dihydroxy-N-(3-hydroxypropyl)-3,3-dimethylbutanamide (D-panthenol, 100 g, 0.488 mol, 1.00 eq) in acetone (1 l).

The solution obtained was then stirred for 3 hours at room temperature, and the pH of the solution was adjusted to 7 with triethylamine. The resulting solution was then concentrated under vacuum, and the residue was applied to a silica gel column with a mixture of petroleum ether and acetone (5.5:1).

65 g (54%) of (4R)-N-(3-hydroxypropyl)-2,2,5,5-tetramethyl-1,3-dioxane-4-carboxamide (compound I) was obtained as a white solid.

LC-MS of compound I: (ES, *m*/*z*):268 [M+Na]⁺, 513 [2M+Na]⁺

### 2. Synthesis of intermediate compound II = DHA ester of compound I

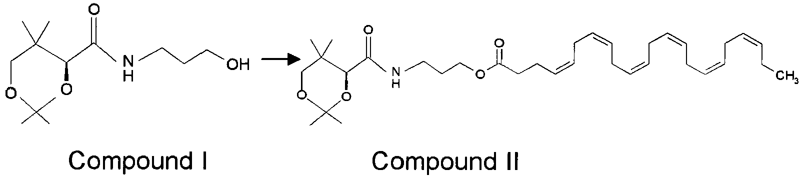

To synthesize this compound, a 1 I three-neck flask purged and maintained under an inert argon atmosphere was used, into which was placed a solution of (4Z, 7Z, 10Z, 13Z, 16Z, 19Z)-docosa-4, 7, 10, 13, 16, 19-hexaenoic acid (CAS 6217-54-5) (70g, 0.213 mol, 1.00 eq), 1',1-[(1H-imidazol-1-yl)carbonyl]-1H-imidazol (51.9 g, 0.320 mol, 1.50 eq), N,N-dimethylpyridin-4-amine (31.2 g, 0.256 mol, 1.2 eq) and (4R)-N-3-hydroxypropyl-2,2,5,5-tetramethyl-1,3-dioxane-4-carboxamide (compound I) (62.7 g, 0.256 mol, 1.20 eq) in dichloromethane (600 ml).

The resulting solution was stirred overnight at room temperature and then diluted with 200 ml of dichloromethane. The resulting solution was then washed with 2x100 ml of water. The organic phase was dried on anhydrous sodium sulfate and concentrated under vacuum. The residue was applied to a silica gel column and eluted by means of a mixture of petroleum ether and acetone (40:1-20:1) so as to obtain 71.0 g (60%) of 3-{[(4R)-2,2,5,5-tetramethyl-1,3-dioxan-4-yl]formamido}propyl(4Z, 7Z, 10Z, 13Z, 16Z, 19Z)-docosa-4, 7, 10, 13, 16, 19-hexaenoate (compound II) as a colorless solid.

### 3. Synthesis of the panthenyl docosahexaenoate of the invention (deprotection of the alcohol functional groups)

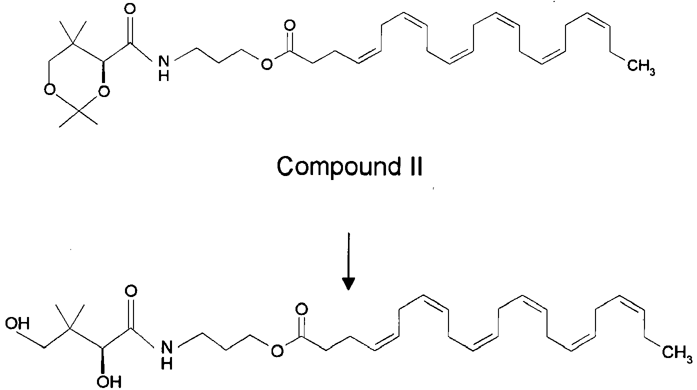

### Compound of formula A of the invention

To synthesize the compound of the invention, a 1 I three-neck flask purged and maintained under an inert argon atmosphere was used, in which was placed a solution of 3-{[(4R)-2,2,5,5-tetramethyl-1,3-dioxan-4-yl]formamido}propyl(4Z,7Z,10Z,13Z,16Z,19Z)-docosa-4,7,10,13,16,19-hexaenoate (compound II) (71 g, 0.128 mol, 1.00 eq) in methanol (710 ml) and p-toluenesulfonic acid (0.972 g, 5.12 mmol, 0.04 eq).

The resulting solution was then stirred overnight at room temperature. The mixture obtained was concentrated under vacuum. The residue was applied to a silica gel column with hexane:acetone (8:1-3:1).

51.9 g (79%) of 3-[(2R)-2,4-dihydroxy-3,3-dimethylbutanamido]propyl(4Z,7Z,10Z,13Z,16Z,19Z)-docosa-4,7,10,13,16,19-hexaenoate (compound of formula A) was obtained as a yellow oil.

LC-MS of the compound of formula A: (ES, m/z): 516 [M+H]⁺, 538 [M+Na]⁺

UPLC of the compound of formula A:
- Column: Waters X-bridge C18, 3.5 µm, 2.1*50 mm
- Mobile phase B: methanol
- Mobile phase A: water/0.05% TFA
- Gradient: from 15% up to 100% of B in 2.3 minutes, 100% B for 1.2 minutes, from 100% up to 15% of B in 0.1 minute, then stop.
- Flow rate: 1.0 ml/min

Chiral HPLC of the compound of formula A: ee%=98.1%
- Column: Chiralpak IA, 0.46*25 cm, 5 µm
- Mobile phase: hexane:ethanol (96:4)
- Flow rate: 1.5 ml/min

HNMR of the compound of formula A: (300 MHz, CDCl₃, ppm):δ 0.92-0.,99 (m, 9H), 1.83-1.90 (m, 2H), 2.07-2.09 (m, 2H), 2.39-2.4 (m, 4H), 2.82-2.85 (m, 10H), 3.31-3.41 (m, 2H), 3.49-3.56 (m, 2H), 4.04 (s, 1 H), 4.14-4.17 (t, J=6.0 Hz, 2H), 5.29-5.42 (m, 12H), 7.02 (s, 1H).

### Example 2: Effect of the panthenyl docosahexaenoate of formula A of the invention on the refractory period in the pig

The beneficial effect of the panthenyl docosahexaenoate of formula A on cardiovascular disease was demonstrated by measuring the atrial refractory period because it is known that an increase in the duration of this parameter is an important event to reduce the onset and the perpetuation of arrhythmias, in particular atrial fibrillation (Attuel *et al.,* 1982; Wijffels *et al.,* 1995).

The study was carried out on male Landrace pigs (20-25 kg). Anesthesia was maintained with isoflurane (0.5-3% of lung tidal volume). Number of respirations and tidal volume were adjusted so as to maintain blood gases within physiological limits.

A left lateral thoracotomy was performed in the fourth intercostal space and the pericardium was opened. Polyethylene-filled catheters were introduced into the nearest thoracic artery to measure arterial pressure during the experiment and in the left saphenous vein to administer the active products or a control carrier.

An atrial electrocardiogram (ECG) was continuously recorded, with three electrodes placed and sutured in the epicardium and the fourth serving as mass and placed in the thoracic muscles. Thus, the ECG provides information on atrial activity. Two bipolar electrodes were also placed in the left atrium at an interval of 0.3 cm and were maintained by fishing hooks. Electrical stimulations were carried out by a stimulator (DS 8000, WPI).

After a sufficient period for the animals to recover from the operation (hemodynamic parameters and blood gases stable and normal), the determination of the refractory period for the animals treated with the active agent or with the control carrier began.

A series of continuous stimuli (S1) was initiated at a rather low voltage (0.1 V), which is insufficient to stimulate the heart, and then the voltage was gradually increased by 0.1 V steps to find the threshold of stimulation which makes it possible to follow the imposed frequency. The search for this threshold was carried out at each stimulation frequency.

Two basic cycle lengths (BCL) of 400 ms and 500 ms were used. Once the threshold was reached, stimulation S1 (train of 10 stimuli) was equal to twice the voltage threshold and extrastimulus S2 was equal to four times the threshold. Every 10 S1, an extrastimulus S2 was initiated during the refractory period (*i.e*., 80 ms after the last S1, the refractory period should in theory last at least 100 ms), and then, every 10 stimuli S1, an extrastimulus was initiated from the last S1 (increments of 5 in 5 ms) until a beat was induced.

The longest interval without a specific response to S2 determines the atrial refractory period (Wirth *et al.,* 2003).

The panthenyl docosahexaenoate of formula A was dissolved in dimethyl acetamide (DMA) and Cremophor^{®} ELP (30/70) diluted 1/4 in glucose (5%). Optionally, a 5% glucose solution was added after ultrasonication.

The panthenyl docosahexaenoate of formula A (quantity: 10 + 10 mg/kg, n=4) was administered in the form of a bolus over 1 minute and then allowed to diffuse for 40 minutes.

The carrier is composed of dimethyl acetamide (DMA) and Cremophor^{®} ELP (30/70) diluted 1/4 in glucose (5%).

The carrier was administered in the same way as the active agent.

The panthenol diester, the isosorbide diester and the ethyl ester were formulated and administered in the same way as the panthenyl docosahexaenoate of formula A.

The ethyl ester is the DHA ethyl ester of the following formula:

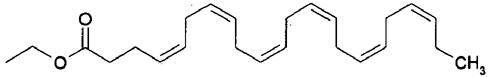

The panthenol diester has the following formula:

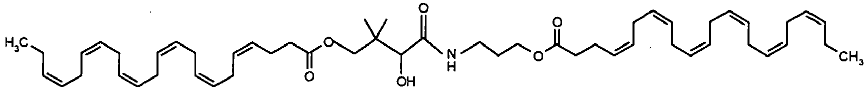

The isosorbide diester has the following formula:

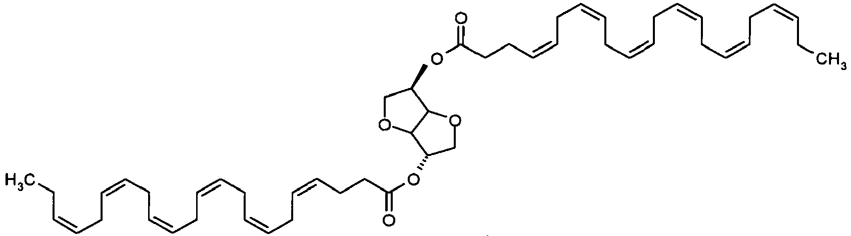

The results are presented in figure 1. This figure represents variations in refractory periods *in vivo* after administration of the carrier versus the panthenyl docosahexaenoate of formula A according to the protocol described above.

The results presented in figure 1 show that the docosahexaenoate panthenyl of formula A significantly increases atrial refractory periods (ARPs) in the treated animals. The intravenous administration of 10 mg/kg + 10 mg/kg of the panthenyl docosahexaenoate of formula A indeed increases the ARPs by 19±2 ms (n=4, p<0.001), while the placebo has no effect whatsoever (-4±3 ms, n=10, NS).

In addition, in a surprising manner, it was noted that the panthenyl docosahexaenoate of formula A is significantly more active than the panthenol diester (in spite of the presence of two DHA molecules per diester molecule), which underscores the significance of the compound of the present invention.

Thus, administration of the panthenyl docosahexaenoate of formula A prolongs the atrial refractory period in the animals of the model and can thus be used to reduce arrhythmia, for example the duration and/or the occurrence of atrial fibrillation (Attuel *et al.,* 1982; Wijffels *et al.,* 1995).

### References

Attuel et al., Failure in the rate adaptation of the atrial refractory period: its relationship to vulnerability, Int J Cardiol. 1982; 2(2): 179-97.
Harrison et al. "Compendium of Synthetic Organic Methods", Vols. 1 to 8 (J. Wiley & Sons, 1971 to 1996).
Wijffels et al., Atrial fibrillation begets atrial fibrillation. A study in awake chronically instrumented goats. Circulation. 1995 Oct. 1; 92(7): 1954-68.
Wirth KJ et al.; Atrial effects of the novel K(+)-channel-blocker AVE0118 in anesthetized pigs. Cardiovasc Res. 2003 Nov. 1; 60(2): 298-306.

## Claims

1. An ester of docosahexaenoic acid with panthenol, of the following formula: 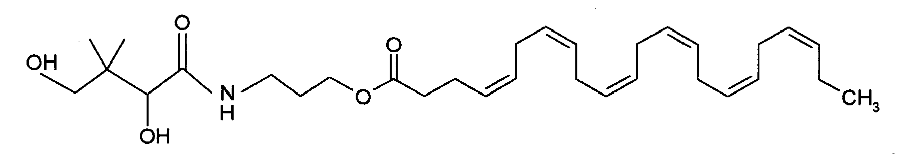 or a pharmaceutically acceptable salt, enantiomer or diastereoisomer of same, or a mixture thereof, including a racemic mixture.

2. The ester of claim 1, of the following formula A:

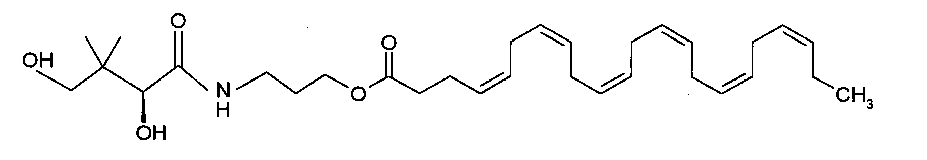

3. The ester of claim 1 or claim 2, for the use of same as a drug.

4. A pharmaceutical composition comprising the ester of claim 1 or claim 2 and a pharmaceutically acceptable excipient.

5. The ester of claim 1 or claim 2 or the composition of claim 4, for the use of same to prevent and/or treat cardiovascular diseases selected from: auricular and/or ventricular arrhythmia, tachycardia and/or fibrillation; to prevent and/or treat diseases represented by defects in electrical conduction in myocardial cells; to prevent and/or treat multiple risk factors for cardiovascular disease selected from: hypertriglyceridemia, hypercholesterolemia, hypertension, in particular refractory arterial hypertension, hyperlipidemia, dyslipidemia, advantageously mixed dyslipidemia; or for primary or secondary prevention and/or treatment of cardiovascular disease derived from auricular and/or ventricular arrhythmia, tachycardia, fibrillation and/or electrical conduction defects induced by myocardial infarction, advantageously sudden death and post-infarction treatment.

6. The ester of claim 5, for the use of same to prevent and/or treat atrial fibrillation.

7. A method for preparing the ester of claim 1 or claim 2, comprising the following steps:
a) Selective protection of two OH functional groups of panthenol or of D-panthenol by an O-protective group, advantageously by trimethylchlorosilane,
b) Esterification of the unprotected OH functional group by DHA in the presence of 1-[(1H-imidazol-1-yl)carbonyl]-1H-imidazol and N,N-dimethylpyridin-4-amine,
c) Deprotection of the two protected OH functional groups.

## Patentansprüche

1. Ester von Docosahexaensäure mit Panthenol gemäß der folgenden Formel: 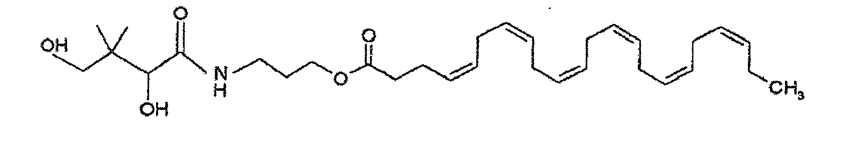 oder ein pharmazeutisch akzeptables Salz, Enantiomer oder Diastereomer desselben, oder eine Mischung hiervon, einschließlich einer razemischen Mischung.

2. Ester nach Anspruch 1 gemäß der folgenden Formel A:

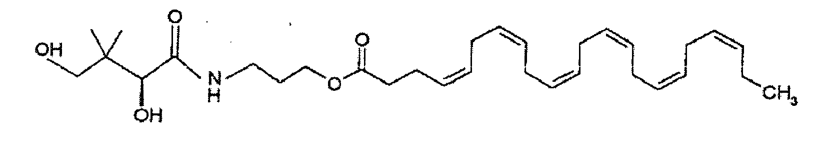

3. Ester nach Anspruch 1 oder 2 für die Verwendung desselben als Arzneistoff.

4. Pharmazeutische Zusammensetzung, umfassend den Ester nach Anspruch 1 oder 2 und einen pharmazeutisch akzeptablen Hilfsstoff.

5. Ester nach Anspruch 1 oder 2 oder Zusammensetzung nach Anspruch 4 für die Verwendung desselben/derselben zur Vorbeugung und/oder Behandlung von kardiovasculären Erkrankungen, ausgewählt aus aurikulärer und/oder ventrikulärer Arrhythmie, Tachykardie oder Fibrillation; zur Vorbeugung und/oder Behandlung von Erkrankungen, die durch Defekte der elektrischen Leitung in myokardialen Zellen gekennzeichnet sind; zur Vorbeugung und/oder Behandlung mehrerer Risikofaktoren für kardiovasculäre Erkrankungen ausgewählt aus Hypertriglyceridämie, Hypercholesterinämie, Bluthochdruck, insbesondere refraktärem arteriellem Bluthochdruck, Hyperlipidämie, Dyslipidämie, vorzugsweise gemischter Dyslipidämie; oder zur primären oder sekundären Vorbeugung und/oder Behandlung von kardiovasculären Erkrankungen, resultierend aus aurikulärer und/oder ventrikulärer Arrhythmie, Tachykardie, Fibrillation und/oder Defekten der elektrischen Leitung induziert durch myokardialen Infarkt, vorzugsweise plötzlicher Tod und Post-Infarkt-Behandlung.

6. Ester nach Anspruch 5 zur Verwendung desselben zur Vorbeugung und/oder Behandlung von Vorhofflimmern.

7. Verfahren zur Herstellung des Esters nach Anspruch 1 oder 2, umfassend die folgenden Schritte:
a) Selektives Schützen von zwei OH-funktionalen Gruppen von Panthenol oder D-Panthenol durch eine O-Schutzgruppe, vorzugsweise durch Trimethylchlorsilan,
b) Verestern der ungeschützten OH-funktionalen Gruppe durch DHA in Gegenwart von 1-[(1H-Imidazol-1-yl)carbonyl]-1H-imidazol und N,N-Dimethylpyridin-4-amin,
c) Entschützen der zwei geschützten OH-funktionalen Gruppen.

## Revendications

1. Ester de l'acide docosahexaénoïque avec du panthénol, répondant à la formule : 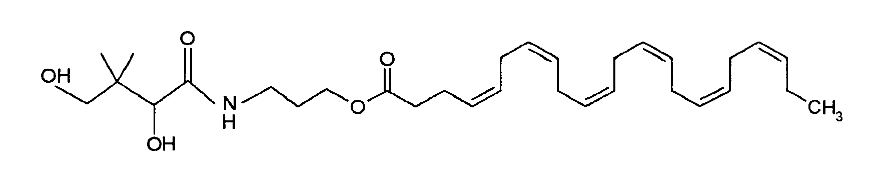 ou un sel pharmaceutiquement acceptable, un énantiomère ou un diastéréoisomère de celui-ci, ou un mélange de ceux-ci, comprenant un mélange racémique.

2. Ester selon la revendication 1, répondant à la formule A :

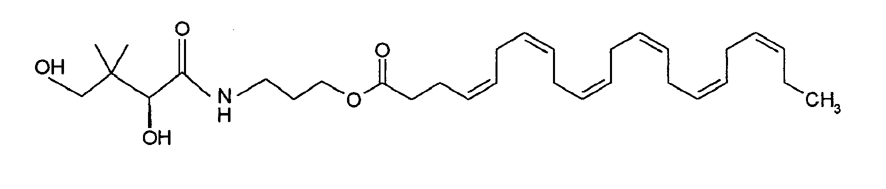

3. Ester selon la revendication 1 ou la revendication 2, pour son utilisation en tant que médicament.

4. Composition pharmaceutique comprenant l'ester selon la revendication 1 ou la revendication 2 et un excipient pharmaceutiquement acceptable.

5. Ester selon la revendication 1 ou la revendication 2 ou la composition selon la revendication 4, pour son utilisation pour la prévention et/ou le traitement de maladies cardiovasculaires choisies parmi : l'arythmie auriculaire et/ou ventriculaire, la tachycardie et/ ou la fibrillation ; pour la prévention et/ou le traitement de maladies représentées par des défauts de conduction électrique dans les cellules myocardiques ; pour la prévention et/ou le traitement de multiples facteurs de risques d'une maladie cardiovasculaire choisis parmi : l'hypertriglycéridémie, l'hypercholestérolémie, l'hypertension, en particulier l'hypertension artérielle réfractaire, l'hyperlipidémie, la dyslipidémie, avantageusement la dyslipidémie mixte ; ou pour la prévention et/ou le traitement primaire ou secondaire d'une maladie cardiovasculaire dérivée de l'arythmie auriculaire et/ou ventriculaire, de la tachycardie, de la fibrillation et/ou de défauts de conduction électrique induits par un infarctus du myocarde, avantageusement un traitement de la mort subite et post-infarctus.

6. Ester selon la revendication 5, pour son utilisation pour la prévention et/ou le traitement de la fibrillation atriale.

7. Procédé de préparation de l'ester selon la revendication 1 ou la revendication 2, comprenant les étapes de :
a) protection sélective de deux groupes fonctionnels OH du panthénol ou du D-panthénol par un groupe O-protecteur, avantageusement par du triméthyl-chlorosilane,
b) estérification du groupe fonctionnel OH non protégé par du DHA en présence de 1-[(1H-imidazol-1-yl)carbonyl]-1H-imidazol et de N,N-diméthylpyridin-4-amine,
c) déprotection des deux groupes fonctionnels OH protégés.
